# EUROPEAN PATENT SPECIFICATION

(11) **EP 1 731 188 B1**
(45) Date of publication and mention of the grant of the patent: **24.10.2012**
(21) Application number: 06252953.2
(22) Date of filing: 07.06.2006
(51) Int. Cl.: A61M 16/08

(54) **Method and device for the automatic identification of respiratory tubes**
Verfahren und Vorrichtung zur selbsttätigen Identifikation von Beatmungsschläuchen
Procédé et dispositif pour l'identification automatique des tubes respiratoires

(30) Priority: 08.06.2005 DE 102005026561
(43) Date of publication of application: 13.12.2006
(73) Proprietor: Dräger Medical GmbH, 23558 Lübeck (DE)
(72) Inventor: Schermeier, Olaf, 23560 Lübeck (DE); Lokotsch, Heiko, 19205 Pokrent (DE); Schoenbeck, Axinja, 23684 Klingberg (DE); Wilkening, Michael, 23568 Lübeck (DE)
(74) Representative: UEXKÜLL & STOLBERG

(56) References cited:
- EP-A- 1 731 089
- EP-A1- 1 188 457
- EP-A2- 0 839 551
- WO-A-00/61003
- DE-U1- 20 113 789
- US-A- 6 126 610
- US-A1- 2003 140 921

## Description

The invention relates to a method for the automatic identification of respiratory tubes.

Modern respiratory systems are characterised by increasing complexity and diversity of possible applications. A plurality of components often has to be connected before a respiratory system is ready for use. Different interfaces usually have to be connected using means of connection, and in some cases this can give rise to an increased risk of error and possibly problems with availability.

An essential connection measure during the configuration of respiratory systems is the coupling-up of the respiratory tube or the respiratory-tube system. The respiratory tube and respiratory-tube system should be regarded in the following as equivalent in connection with the invention, since it does not concern their design in terms of flow characteristics. For convenience this application will refer to respiratory tubes means, the disclosure being equally applicable to respiratory tubes and respiratory tube systems.

It is known in principle to reduce the risk of incorrect connections in medical systems by the fact that identification means are connected to the individual components (DE 201 13 789 U1).

In the area of respiratory systems and respiratory tubes, however, this has been refrained from in the past, since it has been assumed that incorrect connections can be eliminated by the mechanical design of the connectors. The standards and types of respiratory tubes that have since become established, however, give rise to a diversity which cannot be systemised solely by specific types of connector. Each standard type of connector has in the meantime been designed in such a way that it can be connected to different respiratory-tube systems that are intended for very special respiratory modes. This necessitates, however, the precise identification of the type of the connected respiratory-tube system.

For this purpose, various parameters, which are required for optimum functionality of the apparatus and are determined by the tube constitution, have hitherto been measured by the system or inputted by the user into the apparatus software. They include, for example, tube volumes and heating modes.

Document US 6 126 610 discloses a similar identification, specifically for sensor components in the system.

The present invention is as claimed in the claims.

The present invention provides a possible way of reducing the risk of incorrect configurations in respiratory systems and improving operator convenience, without having to incur a greatly increased outlay.

The invention essentially proceeds from the fact that, when a respiratory tube is connected to a respiratory apparatus, connection elements enter into an interaction with one another which forces a relatively precise positioning of one end of the respiratory tube with respect of the respiratory apparatus. This positioning can therefore be used for the positioning of a memory element, which can be read out by a reader unit connected to the respiratory apparatus and which is connected to the respiratory tube. Data permitting an identification of the type of the respiratory tube are stored in the memory element.

The invention consists in a method for the automatic identification of the type of a respiratory tube, wherein a memory element, which is connected to the respiratory tube and on which data are stored that identify the respiratory tube, is read out by a reader unit, which forms a component of the respiratory apparatus, when the respiratory tube is brought into the vicinity of the respiratory apparatus.

To advantage, the identification of the type of the respiratory tube may take place by the fact that the memory element, which is connected to the respiratory tube and on which data are stored that identify the respiratory tube, is read out when the respiratory tube is connected to the respiratory apparatus. Incorrect interpretations due to the reading-out of other data carriers in the vicinity of the reader unit are thus avoided.

To advantage, an alarm may be triggered when a respiratory tube is identified that is not suitable for an intended respiratory mode, or when a respiratory mode is set on the respiratory apparatus that is not suitable for the identified respiratory tube.

At the same time, a check can be made to establish whether the respiratory tube is correctly connected to the respiratory apparatus, and an alarm can be triggered if the respiratory tube is not correctly connected to the respiratory apparatus. Correctly connected within the meaning of the invention is understood to mean the use of an intended respiratory tube and its fitting in a functionable manner. Incorrect positioning can thus largely be eliminated.

It is particularly advantageous for the reader unit to be designed as a writer and reader unit and for additional data to be stored in the memory element connected to the respiratory tube whilst the respiratory tube is connected to the respiratory apparatus. Respiratory parameters, patient data, accounting data, therapy data and/or diagnostic data as well as data recording the use of the respiratory tube, for example, come into consideration as additional data. This includes information concerning cleaning and sterilisation steps.

To advantage, possibilities thus arise of retaining these additional data for subsequent use. After coupling-up of the respiratory tube to the respiratory apparatus, stored respiratory parameters can for example be read out and these respiratory parameters can be adopted by the connected respiratory apparatus, preferably after release given by an authorised member of staff. Data can equally well be processed and relayed to a central system of the hospital logistics or to a patient management system.

An advantage of the invention consists of the fact that, in modern respiratory systems, respiratory tubes are often intended to remain for a lengthy period close to the patient, whereas the actual respiratory apparatuses are often replaced or are connected to the patient only for a short time. By means of the inventive connection of the means for data storage to a respiratory tube that remains for a lengthy period close to the patient, a loss of this data carrier during the time when the respiratory tube is close to the patient is virtually ruled out. The respiratory tube thus assumes, in an inventive way, the function of a patient-related data memory that is always available. The connection of means for data storage to a respiratory tube that remains for a lengthy period close to the patient is automatically associated with the fact that no further manoeuvres are required for preparation of the data carrier and the latter can never be forgotten.

To advantage, the means for reading out the data from the memory element may be integrated into a pneumatic interface on the respiratory apparatus and are designed in such a way that a read-out takes place when a respiratory tube compatible with this interface is connected. The connection means connectable to one another, or more precisely the parts of the pneumatic interface connectable to one another, are connected at least with sufficient dimensional stability to the means for data transmission in a way which ensures that, in the case of the components respiratory tube and respiratory apparatus connected to one another, the means for the data transmission are at least arranged in such a way that a data transmission can take place. This principle of linking means for data transmission to position-fixing connection means connectable to one another is understood as integration within the meaning of the invention.

Furthermore, the effect of integrating the means for data transmission into the pneumatic interface, which in any case has to be connected for the operation of the respiratory system, is that no additional manoeuvres are required for the connection of the means for data storage on the respiratory tube to a writer or reader unit located on the respiratory apparatus, and this greatly assists with a procedure under pressure of time.

It is particularly advantageous for the data transmission to take place contactless, which is advantageous especially when dealing with oxygen to avoid any ignition hazard.

In an advantageous arrangement of the system according to the invention, the means for data storage and/or data transmission are designed in such a way that they are suitable for the storage and transmission of further information via respiratory tubes connectable to the respiratory apparatus. This information can for example include data on production, storage and durability.

In a further advantageous arrangement, the means for data storage and/or data transmission are designed in such a way that they are suitable for the storage and transmission of patient data, therapy data and/or diagnostic data. In this way, the means for data storage can in part assume the function of an electronic patient record and necessary data can automatically be made available to the doctor giving treatment at the time.

It has proved particularly advantageous for the means for data transmission and/or data storage to be components of an RFID system. In particular, the means for data storage include at least one memory element in the form of an RFID tag which is rigidly connected to the respiratory tube.

In order to prevent unauthorised access to the stored data, it is advantageous for the data to be encoded and only to be made available when read out through a suitable decoding process. For this purpose, it is necessary for means of encoding and decoding the transmitted and/or stored data to be contained. These means can for example comprise suitable software components which are integrated into a control unit of the respiratory apparatus.

Furthermore, it is advantageous for means to be contained that make it possible to store manually information which prohibits further use of the respiratory tube connected to the respiratory apparatus. These include, for example, a manual switch which ensures the transmission and storage of a blocking code when actuation takes place. If this code is read out subsequently, the respiratory apparatus requests replacement of the blocked respiratory tube. This can be expedient in the case of unclear infection risks and obvious injuries.

An embodiment of the invention will now be described, by way of example only, with reference to the accompanying Figures, of which:
Fig. 1 is a schematic diagram of a device for the performance of the method according to the invention in the form of a respiratory system,
Fig. 2 is a schematic diagram of such a respiratory system,
Fig. 3 is a schematic diagram of a respiratory system according to the invention in the area of the pneumatic interface, i.e. the coupling-up of the respiratory tube to the respiratory apparatus,
Fig. 4 is a schematic diagram of the general structure of a respiratory system for the performance of the method according to the invention.

The respiratory system equipped according to the invention includes a respiratory tube or a respiratory-tube system with a memory element. Furthermore, respiratory apparatuses are included which have a reader and writer unit, which can communicate with the memory element when the respiratory-tube system is connected. This offers numerous advantages over conventional respiratory systems, as explained in the following.

There are a large number of different types of respiratory tube. Thus, there are single-use and multiple-use tubes, different tube lengths, different diameters, double-tube systems, coaxial tubes, tubes with a semi-permeable membrane for the passage of moisture, tubes heated by electrical heating wires, tubes with temperature sensors and flowmeters.

Many patients are provided with artificial respiration as part of their medical care, whereby different respiratory systems may be used one after the other in the course of the treatment.

Each combination of a specific type of tube with a specific respiratory apparatus requires specific respiratory parameters or excludes other respiratory parameters. Furthermore, respiratory parameters must be selected according to therapeutic aspects. The main parameters are the form of respiration, the oxygen content, the respiratory frequency, where appropriate the stroke volume, the maximum volume, the respiratory pressure and a maximum permissible pressure.

Respiratory systems available at present require that the individual patient respiratory parameters are set manually by the user on each apparatus in order to ensure optimum treatment.

The optimum setting of the parameters depends on a large number of individual patient factors which describe the respiratory requirement. The optimum setting of the respiratory parameters therefore requires a considerable expenditure of time by the staff in attendance.

After the start of the medical care, a patient usually passes through various stations.
These may be: rescue vehicle/helicopter, ambulance, induction, OP, emergence, intensive care unit and various in-patient or outpatient forms of transport. If respiration is required for a patient, the parameters have to be reset by the staff for each respiratory system along this chain in the case of conventional systems.

The described expenditure and the risk of errors are reduced considerably by the use of a respiratory system equipped according to the invention. The type of connected respiratory tube is automatically identified.

Respiratory parameters can be stored as data records in the memory element, which is integrated into the respiratory-tube system. The respiratory-tube system remains with the patient when the clinical area or the respiratory apparatus are changed. After another respiratory apparatus has been connected up, these data are available for the newly connected respiratory apparatus, which enables an automatic or semi-automatic setting of the required respiratory parameters. Furthermore, data can be stored concerning forbidden parameters which must not under any circumstances be set when the given type of tube is used, and this reduces considerably the risk of patients receiving incorrect care. In this way, at least one alarm system can be provided, which triggers an alarm when a respiratory tube is identified which is not suitable for an intended respiratory mode or when a respiratory mode is set on the respiratory apparatus that is not suitable for the identified respiratory tube.

In addition to or as an alternative to the respiratory parameters, data concerning treatment that has taken place can be stored in the memory element and subsequently read out for accounting purposes. For example, the performed respiratory minutes can thus be recorded.

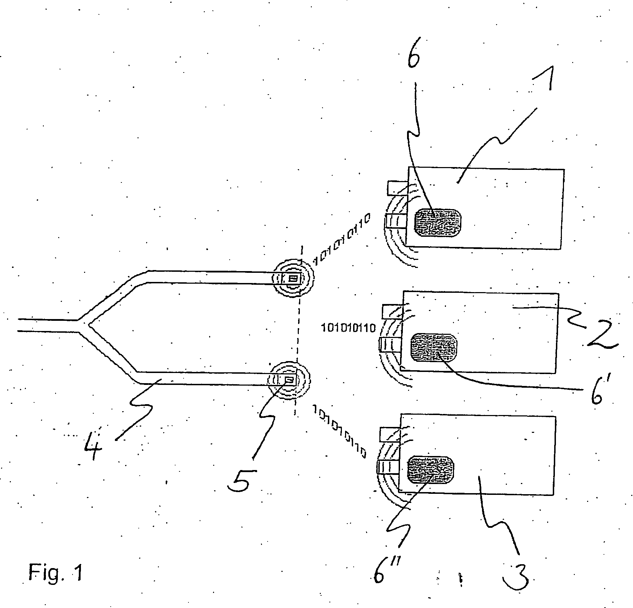

Fig. 1 shows a device for the performance of the method according to the invention in the form of a respiratory system. The example of embodiment concerns a system comprising at least two, in the present case three, respiratory apparatuses 1, 2, 3 and at least one respiratory-tube system 4, whereby the respiratory apparatuses are capable of storing and reading out in a contactless manner individual patient respiratory parameters on a memory element 5 on respiratory-tube system 4 when one of respiratory apparatuses 1, 2, 3 is connected to respiratory-tube system 4. Data enabling identification of the type of connected respiratory-tube system 4 are also stored on memory element 5. The respiratory apparatuses are an emergency respiratory apparatus 1, an intensive-care respiratory apparatus 2 and an anaesthesia respiratory apparatus 3, such as may be used at different times on a patient.

The connection takes place in such a way that respiratory parameters from a respiratory apparatus are stored in each case by a writer and reader unit 6, 6', 6" on memory element 5 of respiratory-tube system 4 and these parameters are read out by the other respiratory apparatuses from memory element 5 in respiratory-tube system 4 and can thus be set automatically or semi-automatically by the individual respiratory apparatuses. The effect of this is that the respiratory parameters set on the first respiratory apparatus, after re-plugging of the respiratory-tube system to another respiratory apparatus, are also set on the latter.

Basic requirements on respiratory tubes are described in EN12342. This standard also defines the mechanical interface to the respiratory system, which is usually designed via a conical male connector on the respiratory system and a female connector on the respiratory tube. The usual standards of 22 mm, 15 mm and 10 mm diameter are available for the connectors. This connector system represents a pneumatic interface within the meaning of the invention, which in the connected state ensures the precise positioning of the shaped pieces in contact with one another.

Each respiratory apparatus automatically stores all the settings of the respiratory parameters on the memory element in the respiratory-tube system. After re-plugging of the tube to another respiratory apparatus, the latter automatically reads out the data last stored on the memory element and sets the latter on the new respiratory apparatus. If necessary, this can take place by retrieval and confirmation on a display screen. If, in turn, any change is made to the settings in this respiratory apparatus, this is automatically stored in the memory element and, when the occasion arises, is transferred to another respiratory apparatus connected up at a later time. In order not to change the process within the clinical procedure, a passive, cableless memory element is used which can be read out without further work steps.

The advantage of the solution for the user lies in the significant simplification of the clinical processes and thus in a reduction in costs as a result of fewer and shorter work steps. The expensive manual patient-specific programming of each individual respiratory apparatus is no longer required and is replaced by a brief retrieval. After a change of the clinical area or the respiratory system, the setting of optimum respiratory parameters can be carried out in a matter of a few seconds, something which in conventional systems takes much more time.

Furthermore, optimum treatment of the patient is ensured in all areas, since incorrect operation of the system is largely eliminated. As a result of the further use of optimised respiratory parameters on different apparatuses, it is possible to achieve a stable and lastingly optimised respiratory state.

The communication between the respiratory-tube system and the respective respiratory apparatus takes place in the example of embodiment via a contactless data connection.

The memory element takes the form of an RFID chip, a so-called tag, in the tube socket. The former is either glued on or injection moulded. It is arranged geometrically in the tube socket in such a way that it can be read out or written on via an antenna by a writer and reader unit in the respiratory apparatus when the respiratory-tube system is connected up to the respiratory apparatus.

RFID performs an inductive process, in which an antenna on a tag is excited at a defined frequency. A small chip on the RFID tag then returns the stored information. There are a large number of different RFID standards and RFID tags with varying functionality.

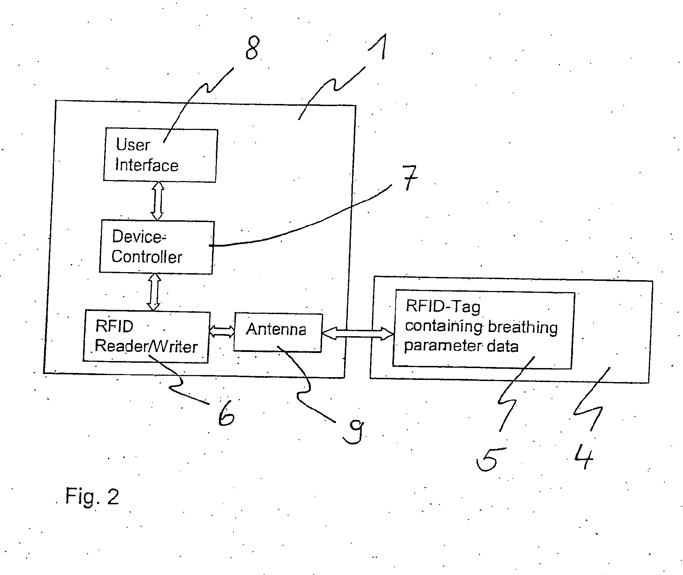

Fig. 2 shows a block diagram of a respiratory system according to the invention. Respiratory apparatus 1 itself contains a control unit 7, which controls all the sequences occurring during the operation of the apparatus. Data required for this can be inputted via an operator unit 8. Respiratory-tube system 4 which can be connected to respiratory apparatus 1 has an RFID tag as a memory element 5. A writer and reader unit 6 in respiratory apparatus 1 can communicate with this RFID tag, which takes place via a suitable antenna 9. Writer and reader unit 6 can also relay data read out from the RFID tag to control unit 7. If the RFID tag contains data concerning respiratory parameters, the latter can replace an input via the operator unit. Instead, the read-in respiratory parameters are displayed at operator unit 8 and accepted as a setting by a release given by the user.

In the as-delivered state of the respiratory-tube system, the RFID tag is already written on with various data identifying the respiratory-tube system. The latter contain information in the form of an identification number and a manufacturer's code and enable the time of manufacture and other specific data to be read out. Furthermore, respiratory parameters are stored which must not be set with the respiratory-tube system. For example, when a respiratory-tube system is being used for neonates, it is thus possible to prevent large stroke volumes being set on the respiratory apparatus that would be typical for respiration in the case of adult patients.

If the respiratory apparatus recognises the RFID tag, this means that a tube is connected. Any respiratory parameters already stored on the RFID tag are then regularly compared with the respiratory parameters set in the apparatus software and, when a change has been made by the user, are stored on the RFID tag. Conversely, after the connection of the respiratory-tube system to a respiratory apparatus, the stored respiratory parameters are read out from the RFID tag via the writer and reader unit and are used automatically or semi-automatically, after release, for the setting of the respiratory mode, which usually takes place through apparatus software.

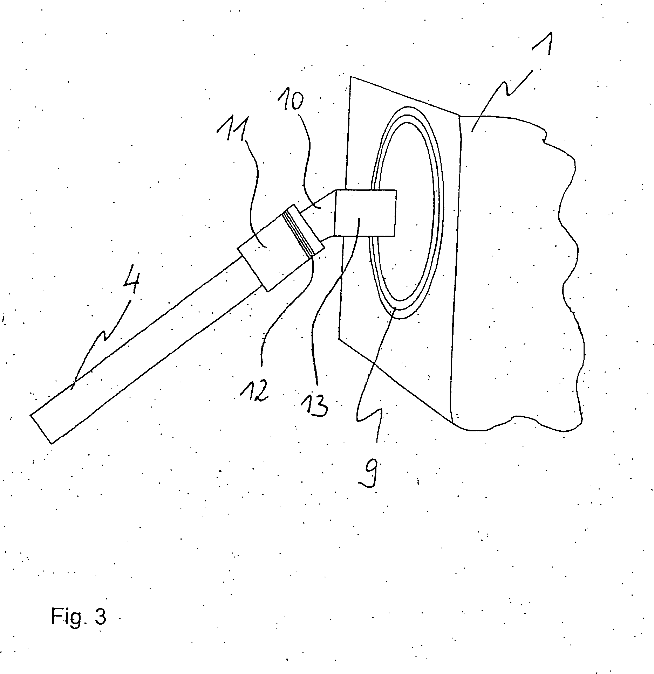

Figure 3 shows a respiratory system according to the invention in the area of the pneumatic interface. A respiratory gas connection with an angle-adjustable male connector 10 is fitted to a respiratory apparatus 1. The connection of a respiratory-tube system 4 is made to this connector 10, whereby a sealing socket 11 as a female connector is connected to male connector 10. An RFID tag, which cannot be seen in the present illustration, is connected to an antenna 12. In the present example, a coil is injection-moulded into socket 11 as antenna 12 of the tag, in such a way that its windings are directed normal to the axis of the tube connection. Antenna 9 of a writer and reader unit on the apparatus side is fitted in this variant at right angles to the axis of the part of respiratory gas connection 13 rigidly connected to respiratory apparatus 1. It thus follows that, with all the positions of the angle-adjustable male connector 10 (except for a connector bent down at right angles), the fields that are formed around antennas 9, 12 exhibit in each case a parallel component with respect to the receiving antenna, which ensures an inductive coupling that is adequate for the performance of the invention.

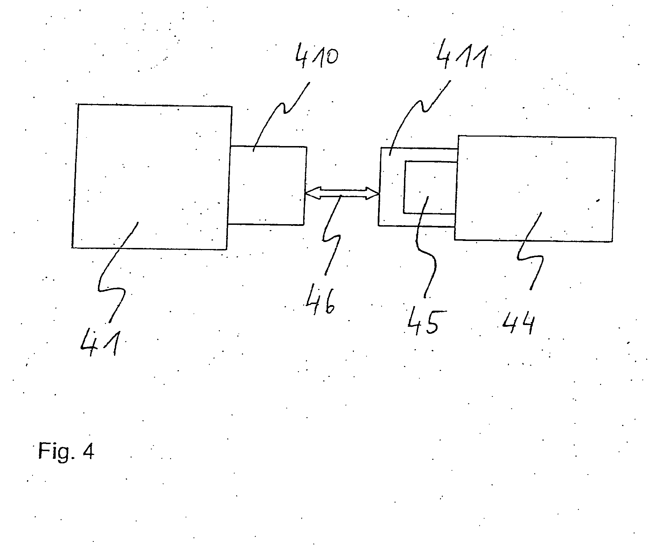

Figure 4 shows the general structure of a respiratory system for the performance of the method according to the invention. This is a respiratory system which includes at least one respiratory-tube system 44 and a respiratory apparatus 41 which can be connected via position-fixing connection means 410, 411, whereby respiratory-tube system 44 contains at least one memory element 45, which can be read out via an interface 46 which is connected mechanically to position-fixing connection means 410, 411.

## Claims

1. A method for the automatic identification of a respiratory tube means, in which a memory element (5), which is connected to the respiratory tube means (4) and on which data are stored which identify the respiratory tube means (4), which data is read out by a reader unit (6), which is a component of a respiratory apparatus (1), when the respiratory tube (4) means is brought into the vicinity of the respiratory apparatus (1).

2. The method according to claim 1, in which the memory element (5) is read out when the respiratory tube (4) means is connected to the respiratory apparatus (1).

3. The method according to claim 1, in which the reader unit (6) is integrated into the pneumatic interface on the respiratory apparatus (1) and the memory element (5) is read out when the respiratory tube (4) means is connected to the respiratory apparatus (1) and compatible with the pneumatic interface which is connected up.

4. The method according to any one of claims 1 to 3, in which after identification of the type of the respiratory tube (4) means, an automatic adaptation of the respiratory mode to the type of the respiratory tube (4) means is performed.

5. The method according to any one of claims 1 to 4, in which an alarm is alarm is triggered when a respiratory tube (4) means is identified that is not suitable for an intended respiratory mode, or when a respiratory mode is set on the respiratory apparatus (1) that is not suitable for the identified respiratory tube (4) means.

6. The method according to any one of claims 1 to 5, in which a check is made to establish whether the respiratory tube (4) means is correctly connected to the respiratory apparatus (1) and an alarm is triggered if the respiratory tube (4) means is not correctly connected to the respiratory apparatus (1).

7. The method according to any one of claims 1 to 6, in which the reader unit (6) is designed as a writer and reader unit and additional data are stored in the memory element (5) connected to the respiratory tube (4) means whilst the respiratory tube (4) means is connected to the respiratory apparatus (1).

8. The method according to claim 7, in which respiratory parameters are stored as additional data.

9. The method according to claim 7 or 8, in which at least patient data, accounting data, therapy data, diagnostic data and/or data recording the use of the respiratory tube (4) means are stored as additional data.

10. The method according to any one of claims 7 to 9, in which, after connection of the respiratory tube (4) means to a respiratory apparatus (1), a read-out of the stored respiratory parameters and adoption of these respiratory parameters by the connected respiratory apparatus (1) takes place.

11. The method according to any one of claims 7 to 10, in which a read-out of stored data takes place and these data are processed and relayed to a central system of the hospital logistics or to a patient management system.

12. The method according to any one of claims 1 to 11, in which the means for data storage and/or data transmission include an RFID system (5, 6, 9, 12).

13. The method according to claim 12, in which, as a memory element (5) on which data are stored that identify the respiratory tube (4), at least one RFID tag is rigidly connected to the respiratory tube (4) means.

14. The method according to any one of claims 1 to 13, in which means (9, 12) for a contactless transmission of data between the respiratory apparatus (1) and the respiratory tube (4) means are integrated into the pneumatic interface (10, 11, 13) between the respiratory apparatus (1) and the respiratory tube (4) means.

15. The method according to any one of claims 1 to 14, in which the transmitted and/or stored data are encoded and decoded.

16. The method according to any one of claims 1 to 15, in which means are contained which make it possible to store manually information which forbids further use of the respiratory tube (4) means.

## Patentansprüche

1. Verfahren zur automatischen Identifizierung einer Beatmungsschlaucheinrichtung, bei dem ein Speicherelement (5), das mit der Beatmungsschlaucheinrichtung (4) verbunden ist und auf dem Daten gespeichert sind, die die Beatmungsschlaucheinrichtung (4) identifizieren, wobei die Daten von einer Leseeinheit (6), die eine Komponente einer Beatmungsvorrichtung (1) ist, ausgelesen werden, wenn die Beatmungsschlaucheinrichtung (4) in die Nähe der Beatmungsvorrichtung (1) gebracht wird.

2. Verfahren nach Anspruch 1, bei dem das Speicherelement (5) ausgelesen wird, wenn die Beatmungsschlaucheinrichtung (4) mit der Beatmungsvorrichtung (1) verbunden wird.

3. Verfahren nach Anspruch 1, bei dem die Leseeinheit (6) in die pneumatische Schnittstelle an der Beatmungsvorrichtung (1) integriert ist und das Speicherelement (5) ausgelesen wird, wenn die Beatmungsschlaucheinrichtung (4) mit der Beatmungsvorrichtung (1) verbunden wird und mit der pneumatischen Schnittstelle, an diese angeschlossen ist, kompatibel ist.

4. Verfahren nach einem der Ansprüche 1 bis 3, bei dem nach der Identifizierung des Typs der Beatmungsschlaucheinrichtung (4) eine automatische Anpassung des Beatmungsmodus an den Typ der Beatmungsschlaucheinrichtung (4) durchgeführt wird.

5. Verfahren nach einem der Ansprüche 1 bis 4, bei dem ein Alarm ausgelöst wird, wenn eine Beatmungsschlaucheinrichtung (4) identifiziert wird, die für den beabsichtigten Beatmungsmodus nicht geeignet ist, oder wenn ein Beatmungsmodus an der Beatmungsvorrichtung (1) eingestellt wird, der für die identifizierte Beatmungsschlaucheinrichtung (4) nicht geeignet ist.

6. Verfahren nach einem der Ansprüche 1 bis 5, bei dem ein Test durchgeführt wird, um festzustellen, ob die Beatmungsschlaucheinrichtung (4) richtig an die Beatmungsvorrichtung (1) angeschlossen ist, und ein Alarm ausgelöst wird, wenn die Beatmungsschlaucheinrichtung (4) nicht richtig an die Beatmungsvorrichtung (1) angeschlossen ist.

7. Verfahren nach einem der Ansprüche 1 bis 6, bei dem die Leseeinheit (6) als Schreib- und Leseeinheit ausgestaltet ist und in dem mit der Beatmungsschlaucheinrichtung (4) verbundenen Speicherelement (5) zusätzliche Daten gespeichert werden, während die Beatmungsschlaucheinrichtung (4) an die Beatmungsvorrichtung (1) angeschlossen ist.

8. Verfahren nach Anspruch 7, bei dem Beatmungsparameter als zusätzliche Daten gespeichert werden.

9. Verfahren nach Anspruch 7 oder 8, bei dem wenigstens Patientendaten, Buchführungsdaten, Therapiedaten, Diagnosedaten und/oder Daten, die die Verwendung der Beatmungsschlaucheinrichtung (4) aufzeichnen, als zusätzliche Daten gespeichert werden.

10. Verfahren nach einem der Ansprüche 7 bis 9, bei dem, nach Anschluss der Beatmungsschlaucheinrichtung (4) an die Beatmungsvorrichtung (1), die Auslesung von Beatmungsparametern und die Übernahme dieser Beatmungsparameter durch die angeschlossene Beatmungsvorrichtung (1) stattfinden.

11. Verfahren nach einem der Ansprüche 7 bis 10, bei dem eine Auslesung gespeicherter Daten stattfindet und diese Daten bearbeitet werden und zu einem zentralen System der Krankenhauslogistik oder zu einem Patienten-Managementsystem weitergeleitet werden.

12. Verfahren nach einem der Ansprüche 1 bis 11, bei dem die Einrichtungen zur Datenspeicherung und/oder Datenübertragung ein RFID-System (5, 6, 9, 12) enthalten.

13. Verfahren nach Anspruch 12, bei dem als Speicherelement (5), auf dem den Beatmungsschlauch (4) identifizierende Daten gespeichert sind, wenigstens ein RFID-Tag fest mit der Beatmungsschlaucheinrichtung (4) verbunden ist.

14. Verfahren nach einem der Ansprüche 1 bis 13, bei dem Mittel (9, 12) zum kontaktlosen Übertragen von Daten zwischen der Beatmungsvorrichtung (1) und der Beatmungsschlaucheinrichtung (4) in die pneumatische Schnittstelle (10, 11, 13) zwischen der Beatmungsvorrichtung (1) und der Beatmungsschlaucheinrichtung (4) integriert sind.

15. Verfahren nach einem der Ansprüche 1 bis 14, bei dem die übertragenen und/oder gespeicherten Daten kodiert und dekodiert werden.

16. Verfahren nach einem der Ansprüche 1 bis 15, bei dem Mittel vorhanden sind, die es ermöglichen, manuell Informationen zu speichern, die die weitere Verwendung der Beatmungsschlaucheinrichtung (4) verbieten.

## Revendications

1. Procédé pour l'identification automatique d'un tube respiratoire dans lequel un élément de mémoire (5), qui est relié au tube respiratoire (4) et sur lequel des données qui identifient le tube respiratoire (4) sont sauvegardées, élément de mémoire dont les données sont lues par une unité de lecture (6) qui est un composant d'un appareil respiratoire (1) lorsque le tube respiratoire (4) est amené à proximité de l'appareil respiratoire (1).

2. Procédé selon la revendication 1 dans lequel l'élément de mémoire (5) est lu lorsque le tube respiratoire (4) est relié à l'appareil respiratoire (1).

3. Procédé selon la revendication 1 dans lequel l'unité de lecture (6) est intégrée à l'interface pneumatique sur l'appareil respiratoire (1) et l'élément de mémoire (5) est lu lorsque le tube respiratoire (4) est relié à l'appareil respiratoire (1) et compatible avec l'interface pneumatique qui est reliée.

4. Procédé selon l'une quelconque des revendications 1 à 3 dans lequel, après identification du type de tube respiratoire (4), une adaptation automatique du mode respiratoire au type de tube respiratoire (4) est réalisée.

5. Procédé selon l'une quelconque des revendications 1 à 4 dans lequel une alarme est déclenchée lorsqu'un tube respiratoire (4) est identifié qui n'est pas approprié pour un mode respiratoire visé ou lorsqu'un mode respiratoire qui n'est pas approprié pour le tube respiratoire identifié (4) est réglé sur l'appareil respiratoire (1).

6. Procédé selon l'une quelconque des revendications 1 à 5 dans lequel un contrôle est fait pour établir si le tube respiratoire (4) est relié correctement à l'appareil respiratoire (1) et une alarme est déclenchée si le tube respiratoire (4) n'est pas relié correctement à l'appareil respiratoire (1).

7. Procédé selon l'une quelconque des revendications 1 à 6 dans lequel l'unité de lecture (6) est conçue comme une unité d'enregistrement et de lecture et des données additionnelles sont sauvegardées dans l'élément de mémoire (5) relié au tube respiratoire (4) tandis que le tube respiratoire (4) est relié à l'appareil respiratoire (1).

8. Procédé selon la revendication 7 dans lequel des paramètres respiratoires sont sauvegardés comme données additionnelles.

9. Procédé selon la revendication 7 ou 8 dans lequel au moins des données de patient, des données comptables, des données de thérapie, des données de diagnostic et/ou des données enregistrant l'utilisation du tube respiratoire (4) sont sauvegardées comme données additionnelles.

10. Procédé selon l'une quelconque des revendications 7 à 9 dans lequel, après connexion du tube respiratoire (4) à l'appareil respiratoire (1), une lecture des paramètres respiratoires sauvegardés et l'adoption de ces paramètres respiratoires par l'appareil respiratoire connecté (1) a lieu.

11. Procédé selon l'une quelconque des revendications 7 à 10 dans lequel une lecture des données sauvegardées a lieu et ces données sont traitées et transmises à un système central de logistique hospitalière ou à un système de gestion de patients.

12. Procédé selon l'une quelconque des revendications 1 à 11 dans lequel les moyens pour la sauvegarde de données et/ou la transmission de données comprennent un système RFID (5, 6, 9, 12).

13. Procédé selon la revendication 12 dans lequel au moins une étiquette RFID en tant qu'élément de mémoire (5), sur lequel des données qui identifient le tube respiratoire (4) sont sauvegardées, est reliée de manière fixe au tube respiratoire (4).

14. Procédé selon l'une quelconque des revendications 1 à 13 dans lequel les moyens (9,12) pour une transmission sans contact de données entre l'appareil respiratoire (1) et le tube respiratoire (4) sont intégrés dans l'interface pneumatique (10, 11, 13) entre l'appareil respiratoire (1) et le tube respiratoire (4).

15. Procédé selon l'une quelconque des revendications 1 à 14 dans lequel les données transmises et/ou sauvegardées sont codées et décodées.

16. Procédé selon l'une quelconque des revendications 1 à 15 dans lequel des moyens sont contenus qui rendent possible de sauvegarder manuellement l'information qui empêche la poursuite de l'utilisation du tube respiratoire (4).
